(19) Europäisches Patentamt
European Patent Office
Office européen des brevets

(11) Publication number: **0 397 650 B1**

(12) **EUROPEAN PATENT SPECIFICATION**

(45) Date of publication of patent specification: **27.10.93**

(51) Int. Cl.5: **B01J 23/60**, B01J 37/00, C07C 29/14, C07C 29/136

(21) Application number: **88908047.9**

(22) Date of filing: **29.07.88**

(86) International application number: **PCT/US88/02576**

(87) International publication number: **WO 89/00886 (09.02.89 89/04)**

# (54) LOW PRESSURE CATALYTIC HYDROGENATION OF CARBONYL-CONTAINING COMPOUNDS AND CATALYSTS THEREFOR.

(30) Priority: **03.08.87 US 81252**
**01.02.88 US 150791**
**15.07.88 US 219630**

(43) Date of publication of application: **22.11.90 Bulletin 90/47**

(45) Publication of the grant of the patent: **27.10.93 Bulletin 93/43**

(84) Designated Contracting States: **DE FR GB IT NL**

(56) References cited:
**EP-A- 0 241 760**
**FR-A- 1 392 376**
**US-A- 2 818 393**
**US-A- 4 052 467**

(73) Proprietor: **EASTMAN KODAK COMPANY**
**343 State Street**
**Rochester, New York 14650-2201(US)**

(72) Inventor: **GUSTAFSON, Bruce Leroy**
**831 Sir Echo Drive**
**Kingsport, TN 37663(US)**
Inventor: **WEHNER, Paul Sherman**
**4017 Lakewood Drive**
**Kingsport, TN 37663(US)**
Inventor: **MERCER, Patricia Lee Nixon**
**5039 Dublin Road**
**Kingsport, TN 37664(US)**
Inventor: **NELSON, Gregory, Otis**
**1136 Olympus Drive**
**Kingsport, TN 37663(US)**

(74) Representative: **Buff, Michel et al**
**Kodak-Pathé**
**Département des Brevets et Licences CRT**
**Centre de Recherches et de Technologie**
**Zone Industrielle**
**F-71102 Chalon sur Saône Cédex (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 397 650 B1

## Description

This invention relates to catalytic hydrogenation. In one aspect, the present invention relates to a process for the selective reduction of carbonyl-containing compounds to alcohols. In another aspect, the present invention relates to catalysts useful for the selective reduction of carbonyl-containing compounds to alcohols, and methods for preparing such catalysts.

The catalytic hydrogenation of carbonyl-containing compounds, e.g., esters, to produce their corresponding alcohols, is potentially of great commercial value. Catalysts traditionally employed for such conversions include copper chromite based materials, frequently containing a promoter such as barium. Unfortunately, these catalysts typically require high pressure to achieve commercially attractive reaction rates for the hydrogenation of esters, i.e., pressures in excess of 3000 psig (21000 kPa). In addition chromium and barium present toxicity and environmental concerns which must be dealt with if one is to economically and safely use these materials on a commercial scale.

More recently, substantial amounts of research have been carried out in efforts to develop hydrogenation catalysts capable of reducing carbonyl-containing compounds, e.g., organic acids and esters, to alcohols at reduced pressures. While such catalysts are capable of promoting the hydrogenation of carbonyl-containing compounds to produce alcohols, one problem with such materials is the need to run at very low liquid hourly space velocities in order to achieve suitably high conversion levels.

Another problem frequently encountered with such prior art low pressure catalyst systems when employed for the reduction of carbonyl-containing compounds such as aldehydes and ketones, is their lack of selectivity to the desired alcohol product, such catalysts frequently being too active and thus producing product which results from reaction of substrate with additional hydrogen.

Yet another problem encountered with such prior art low pressure catalyst systems, such as Raney nickel, is the ease of handling of such catalysts, which are frequently pyrophoric, and thus require special handling to avoid fire hazard.

### Objects of the Invention

An object of the present invention, therefore, is a process for the low pressure, high selectivity, high activity hydrogenation of carbonyl-containing compounds to produce alcohols.

Another object of the present invention is a catalyst system which is capable of promoting the hydrogenation of carbonyl-containing compounds at low reaction pressures.

Still another object of the present invention is a catalyst system which is capable of promoting the hydrogenation of carbonyl-containing compounds at low reaction pressure, which catalyst system is readily prepared and requires no special handling precautions.

These and other objects of the present invention will become apparent from inspection of the detailed description and the appended claims which follow.

### Statement of the Invention

In accordance with the present invention, it has been discovered that palladium and zinc supported on a carrier is an effective catalyst for the low pressure hydrogenation of carbonyl-containing compounds to selectively produce alcohols in high yield.

In accordance with another embodiment of the present invention, it has been discovered that a catalyst prepared by first depositing palladium on a support, then reducing the palladium sufficiently to "stabilize" the palladium on the support, and then contacting the palladium-treated support with zinc is exceptionally effective for the low pressure hydrogenation of carbonyl-containing compounds to selectively produce alcohols in high yield.

The invention hydrogenation process employs readily prepared, easily handled catalysts and enables a commercially important reaction, i.e., the conversion of carbonyl-containing compounds to alcohols, to be carried out at low reaction pressures, thereby reducing the cost of equipment required for the desired hydrogenation reaction and reducing the safety risks involved in such conversions.

### Detailed Description of the Invention

In accordance with the present invention there is provided a method for preparing high activity, low pressure hydrogenation catalysts comprising palladium and zinc on a support, which method comprises:

(a) contacting said support with at least one of zinc or a reducible compound thereof and palladium or a reducible compound thereof;

(b) optionally calcining the resulting palladium and/or zinc treated support in the presence of an oxygen-containing gas at a temperature in the range of 200 up to 400 °C for a time sufficient to activate said palladium and/or said zinc component;

(c) where palladium is employed in step (a), optionally contacting said palladium-treated support with a reducing atmosphere under conditions sufficient to cause reduction of at least a portion of the palladium to less than the +2 oxidation state ;

(d) optionally contacting said support again with at least one member selected from the group consisting of palladium or a reducible compound thereof and zinc or a reducible compound thereof ; with the proviso that said support is ultimately contacted with both palladium and zinc ;

(e) optionally calcining the resulting palladium and zinc treated support in the presence of an oxygen-containing gas at a temperature in the range of 200° up to 400 °C for a time sufficient to activate said palladium and said zinc component ; and thereafter

(f) contacting the zinc and palladium-treated support with a reducing atmosphere under conditions sufficient to cause reduction of at least a portion of the palladium to less than the +2 oxidation state.

As suggested by the above-described method for catalyst preparation, support can be contacted simultaneously with appropriate palladium and/or zinc components ; or support can be contacted with one component followed by a subsequent contacting with the other component ; or support can be treated via multiple contacting (with optional calcination/reduction treatments between contacting) with one or both of the palladium and zinc catalyst components. Whichever option is selected, the method of the invention provides a catalyst comprised of a support bearing zinc and palladium with the above-mentioned oxidation state.

In accordance with the present invention there is further provided a presently preferred method for preparing supported high activity, low pressure hydrogenation catalysts comprising palladium and zinc on a support, which method comprises:

(a) contacting said support with palladium or a reducible compound thereof;

(b) contacting said palladium-treated support with a reducing atmosphere under conditions sufficient to form highly dispersed, supported zerovalent palladium;

(c) contacting said supported zerovalent palladium with zinc or a reducible compound thereof; and thereafter

(d) optionally calcining the resulting palladium and zinc treated support in the presence of an oxygen-containing gas at a temperature in the range of 200° up to 400 °C for a time sufficient to remove undesired counter-ions and/or ligands, thereby activating said zinc component.

Catalysts employed in another embodiment of the present invention comprise palladium and zinc on a carrier or support. A wide variety of techniques for contacting support with palladium and zinc are contemplated for use in the practice of the present invention. For example, palladium or a palladium precursor can be applied directly to support employing such techniques as incipient wetness, wet impregnation, ion exchange, metal atom evaporation, precipitation; then zinc or a zinc precursor can be similarly applied to support; or support can be contacted with both zinc and palladium at the same time; or appropriate precursors of palladium and zinc can be coprecipitated in the presence of support, and then calcined and/or reduced to convert the palladium and zinc to an active form.

In accordance with he presently preferred method for preparing supported catalyst, after support has been contacted with palladium or a palladium precursor, and before the support is further treated with zinc, the palladium-treated support is subjected to reducing conditions sufficient to fix the palladium on the support in a highly dispersed and reduced state so that redispersion of the palladium does not occur upon exposure to corrosive zinc species such as nitrates. For example, this can be accomplished by subjecting the palladium-treated support under a hydrogen atmosphere at a temperature in the range of 25 up to 400 °C.

When supported catalyst is contemplated for use in the practice of the invention process, a wide range of inorganic materials can be employed as support for the invention Pd/Zn catalyst. Exemplary materials are relatively non-acidic in that they do not promote significant levels of such undesired side reaction as transesterification, alcohol dehydration, ester hydrolysis, and the like. Such materials include silica ($SiO_2$), titania ($TiO_2$), carbon (C), rare earth oxides (e.g., $La_2O_3$, $CeO_2$) and alumina ($Al_2O_3$), as well as mixtures of any two or more thereof.

The surface area of the catalyst supports employed in the practice of the present invention can vary widely. Preferably, support materials employed in the practice of the invention will have surface areas of at least about 1 $m^2/g$. Of course, those of skill in the art also recognize that higher surface area materials will

generally produce higher activity catalysts than lower surface area catalysts having comparable composition.

Suitable sources of palladium are any compounds which are reducible when subjected to reducing conditions. Since many palladium compounds are convertible to the oxide form upon calcination under the above-described conditions, and the oxides of palladium are readily reduced, many palladium compounds are useful for catalyst preparation. Exemplary palladium compounds include the palladium halides, palladium acetate, palladium nitrate, palladium ammine complexes and organometallic complexes of palladium.

When the palladium and/or zinc components of the invention supported catalyst are provided as precursor moieties, it is preferred to subject the Pd/Zn-treated support to a calcination treatment at temperatures in the range of 200 up to 400 °C. Such temperature is maintained for a time sufficient to activate the zinc and the palladium components used to form the catalyst. Times in the range of 2 up to 8 hours or longer are generally effective for this purpose.

Finally, the Pd/Zn-treated support can optionally be subjected to a reducing atmosphere under conditions sufficient to cause reduction of at least a portion of the palladium to less than the +2 oxidation state.

When the zinc component of the presently preferred supported catalyst (i.e., where palladium is "fixed" on the support before zinc is applied) is provided as a precursor moiety, it is preferred to subject the Pd/Zn-treated support to a calcination treatment at temperatures in the range of 200 up to 400 °C. Such temperature is maintained for a time sufficient to remove undesired counter-ions and/or ligands, thereby activating the zinc component used to form the catalyst. Times in the range of 2 up to 8 hours or longer are generally effective for this purpose.

The term "carbonyl-containing compounds" as employed in this specification is intended to include compounds of the structure

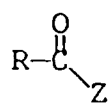

wherein R is a $C_1$-$C_{20}$ alkyl or substituted alkyl radical; or

a $C_2$-$C_{20}$ alkenyl or alkynyl radical or substituted derivative thereof;

wherein said substituted groups include ethers, amines, additional carbonyl groups, aryl groups, hydroxyl groups and alkoxy groups; and

Z = H,

R', wherein R' is defined the same as R, and is selected independently of R,

OR', wherein R' is as defined above,

X, wherein X is any one of the halogens, or

$NR''_2$, wherein each R'' is independently selected from H or R';

with the proviso that R and Z can be joined as part of a polymethylene or hydrocarbyl- or heteroatom-substituted polymethylene radical,

poly-carbonyl analogs of such carbonyl-containing compounds; and

mixtures of any two or more thereof.

Preferred carbonyl-containing compounds are compounds selected from the group consisting of:

(a) $YO_2C—A—CO_2Y$ ,

wherein A is an alkylene moiety, an alkenylene moiety, or an alkynylene moiety having 1 up to 20 carbon atoms, or substituted derivative thereof, or a cycloalkyl or cycloalkenyl moiety having 4-12 carbon atoms or substituted derivative thereof; and wherein each Y is independently a $C_1$ up to $C_{12}$ alkyl, alkenyl or alkynyl radical or substituted derivative thereof;

(b) $B$-$CO_2Y$

wherein B is an alkyl, alkenyl or alkynyl radical, or substituted derivative thereof, having 1 up to 20 carbon atoms; and

wherein Y is defined as above;

(c) 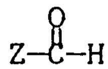

wherein Z is an alkyl, alkenyl or alkynyl radical having 1 up to 20 carbon atoms or substituted derivatives thereof; and

mixtures of any two or more thereof.

Exemplary carbonyl-containing compounds which satisfy the above formulae include alkyl oleates, dialkyl adipates, propionaldehyde, dialkyl cyclohexane dicarboxylates, alkyl acrylates, alkyl propionates, alkyl isobutyrates, alkyl normal butyrates, alkyl acetates, nonanal, dialkyl butane dicarboxylates, alkyl methacrylates, alkyl crotonates, alkyl isocrotonates, alkyl sorbates, alkyl cinnamates, maleic anhydride, alkyl fumarates, dialkyl succinates, succinic anhydride, alkyl glutarates, dialkyl malonates, dialkyl octanedioates, dialkyl decanedioates, dialkyl dodecanedioates, alkyl laurates, alkyl myristates, alkyl palmitates, alkyl stearates, alkyl linoleates, alkyl linolenates, alkyl isovalerates, alkyl normal valerates, alkyl caproates, alkyl caprylates, alkyl 2-ethylhexanoates, dialkyl cyclohexanedioates, $\gamma$-butyrolactone, alkyl phenylacetates, alkyl cyclohexane carboxylates, alkyl pyruvates, alkyl glycolates, alkyl oxalates, alkyl formates, alkyl lactates, alkyl citrates and glyceride esters.

Typical alkyl groups employed have from 1 up to 20 carbon atoms, with alkyl groups having 1 up to 6 carbon atoms being preferred.

The hydrogenation process of the present invention involves contacting at least one of the above-described carbonyl-containing compounds with at least one of the above-described palladium catalysts in the presence of hydrogen under hydrogenation condtions. Hydrogenation conditions typically employed in the practice of the present invention are set forth below.

The process of the present invention can be operated in a variety of configurations. Depending on the substrate to be hydrogenated, the preferred method of opeation is frequently in a fixed bed flow reaction system. If the vapor pressure of the substrate to be hydrogenated is sufficiently high at reaction temperature, the desired method of operation may be vapor phase, i.e., all reactants and products exist in the gaseous phase. For other substrates, the desired method of operation may be a trickle bed configuration. Regardless of the method of operation, the desired time of contact between the reactants and catalyst components can be varied as desired to achieve the desired level of reaction.

In typical fixed bed operation, pressures in the range of 100-10,000 psig (700 - 70000 kPa) will be employed. Preferably, the pressure will be in the range of 100-2500 psig (700 - 17500 kPa). Similarly, temperatures in the range of 25-400 °C can be used, with a more perferred range of 100-290 °C. While the feed rate of the substrate will be varied to control the level of conversion, normal liquid hourly space velocities (LHSV) will be in the range of 0.01-100 $h^{-1}$, with a preferred range of 0.1-20 $h^{-1}$. The molar ratio of hydrogen to substrate will typically be in the range of 1:1 to 1000:1 with a preferred range of 2:1 to 100:1.

Alternatively the invention may be conducted in a slurry phase reactor. In slurry phase operation, the ratio of carbonyl-containing compound to catalysts employed can vary widely, with ratios as low as 1:1 or lower being operable, but not economically attractive; and ratios as high as 10,000:1 and higher also being operable, but generally providing relatively low conversions unless very long contact times are employed. Preferred carbonyl-containing compound:catalysts ratios fall within the range of 1:1 up to 1,000:1, with ratios in the range of 2:1 up to 100:1 being most preferred because good levels of conversion of the carbonyl-containing compounds are obtained without requiring excessive amounts of catalysts, or extremely long contact times.

While the invention hydrogenation process can be carried out in the absence of solvent, it is presently preferred to perform the process in the presence of a suitable solvent. Suitable solvents are compounds which are fluid and in which the carbonyl-containing starting material is soluble at reaction temperature, and which are non--reactive under hydrogenation conditions. Preferred solvents are those which are fluid and in which the carbonyl-containing starting material is soluble at room temperature. Exemplary solvents include aromatic solvents such as toluene; alcohols such as methanol; ethers such as diphenyl ether and tetrahydrofuran.

When employed, the volume/volume ratio of solvent to substrate can vary widely, typically falling in the range of 5:95 to 95:5.

In a preferred embodiment of the present invention, hydrogenation of carbonyl-containing compounds is carried out with small amounts of water (i.e., 0.01 up to 2 wt. % water based on the total weight of reactants and solvent) present in the reaction mixture. It has been found that selectivity to hydrogenation (as opposed to transesterification between reactant and product) products is greatly improved by the presence of such small quantities of water in the reaction mixture.

Following hydrogenation, the desired product can be recovered and purified using conventional techniques well known to those of skill in the art. For example, catalysts can be removed from the reaction mixture by filtration, decantation and the like. By-products and unreacted starting material as well as solvent, if employed, can be separated from the product by distillation, recrystallization and solvent/solvent extraction.

The invention will now be described in greater detail by reference to the following non-limiting examples.

EXAMPLE 1 - Catalyst Preparation

b. Pd-Zn on $SiO_2$ Support

A sample of 1 wt % Pd on Davison Grade 59 $SiO_2$ was prepared by dissolving 0.48 grams (g) of $Pd(NO_3)_2$ in 29.4 mL of water and adding the resulting solution to about 20 g of the solid $SiO_2$ support material. The resulting solid sample was dried in air at 90 °C for 30 minutes and then further calcined in air at 350 °C for 12 hours. This resulting $Pd/SiO_2$ catalyst was split into separate fractions for zinc addition. Zinc was added by dissolving the appropriate amount of zinc nitrate in a small amount of water and adding this solution to the $Pd/SiO_2$ sample. The resulting solid was dried at 90 °C in air and then calcined at 200 °C for several hours prior to evaluation for hydrogenation of carbonyl-containing compounds.

c. Pd-Zn on $SiO_2$ with Intervening Reduction of Pd

A sample of 2.8 wt % Pd on Davison Grade 59 $SiO_2$ was prepared by dissolving 7 grams (g) of $[Pd(NH_3)_4]Cl_2$ in 25 mL of water and adding the resulting solution to about 100 g of the $SiO_2$ support material which was suspended in about 2,000 mL of water containing sufficient $NH_4OH$ to adjust the pH to about 11.0. After soaking for about two hours at room temperature, the $SiO_2$ was recovered by filtration, washed with about 400 mL of water, then dried in air at 120 °C for about 18 hours and then further treated to reducing conditions as follows:

(a) 500 standard cubic centimeters per minute (sccm) of argon for about 1 hour at room temperature;
(b) 500 sccm of argon for an additional 1 hour at 120 °C;
(c) 500 sccm of argon for an additional 1 hour at 260 °C;
(d) argon flow was replaced with hydrogen and catalyst temperature increased; hydrogen flow was maintained for about 1.5 hours after the temperature had reached about 400 °C; then
(e) the catalyst was allowed to cool to about 380 °C, the hydrogen flow replaced with about 500 sccm of argon, and catalyst then allowed to cool to room temperature under continued argon flow.

The resulting $Pd/SiO_2$ catalyst was split into separate fractions for zinc addition. Zinc was added by dissolving the appropriate amount of zinc nitrate $[Zn(NO_3)_2\text{-}6H_2O]$ in about 50 mL of water and adding this solution to 20 g portions of the $Pd/SiO_2$ sample. The resulting solid was dried initially by stirring to steam bath temperature, followed by heating to about 120 °C in air and then calcined in flowing air at 200 °C for 3 hours prior to evaluation for hydrogenation of carbonyl-containing compounds.

EXAMPLE 3 - Hydrogenation of Methyl Acetate

b. Supported Pd-Zn Catalysts

Catalysts prepared as described in Example 1b and 1c were employed for the vapor phase hydrogenation of methyl acetate.

Catalytic evaluations were conducted in a vapor chase micro-reactor system using 1-2 cc of powdered catalyst. Methyl acetate feed was pumped into a heated chamber which was purged with the hydrogen feed gas. Typical GHSV were in the range of 30,000 $h^{-1}$, with $H_2$/ester ratios in the range of 3.6 up to 4.5 being typical. Product analysis of exit stream from the reactor was accomplished by gas chromatography. Results obtained at 300 °C, 750 psig (5250 kPa), at conversions of <20% are presented in Table III.

## Table III

### Methyl Acetate Hydrogenolysis

2.8% Pd–Zn/$SiO_2$ Catalyst
300°C
700 psig (7900 kPa)
$H_2$ /Ester = 3.0

| Catalyst | Rate (µmole/g–cat sec) MeOH | EtOH | EtOAc | Total* |
|---|---|---|---|---|
| A. Supported Catalyst Prepared as described in Example 1b: | | | | |
| Pd/$SiO_2$ | 1.21 | 0.016 | 0.073 | 0.089 |
| Pd–Zn/$SiO_2$ (1:1)** | 2.74 | 0.024 | 0.85 | 0.87 |
| Pd–Zn/$SiO_2$ (1:2)** | 6.48 | 0.12 | 2.25 | 2.37 |
| Pd–Zn/$SiO_2$ (1:5)** | 3.09 | 0.00 | 0.84 | 0.84 |
| Pd–Zn/$SiO_2$ (1:10)** | 2.31 | 0.012 | 0.68 | 0.69 |
| Zn/$SiO_2$ | 0.00 | 0.00 | 0.00 | 0.00 |
| B. Supported Catalyst Prepared as Described in Example 1c: | | | | |
| Pd–Zn (1:0) | 21. | 3.5 | 2.1 | 5.6 |
| Pd–Zn (1:1) | 46. | 24. | 14. | 38. |
| Pd–Zn (1:2) | 48. | 16. | 19. | 35. |
| Pd–Zn (1:5) | 6.7 | 0.7 | 2.8 | 3.5 |
| Pd–Zn (1:10) | 2.6 | .08 | 1.1 | 1.2 |

Table III (Cont'd)

| Catalyst | Rate (μmole/g-cat sec) MeOH | EtOH | EtOAc | Total* |
|---|---|---|---|---|
| Pd-Zn (1:0) | 0.0 | 0.0 | 0.0 | 0.0 |
| Comparative Example*** Pd-Zn (1:2) | 8.7 | 3.7 | 3.1 | 6.8 |

MeOH - Methanol, EtOH = Ethanol, EtOAc = Ethyl Acetate

*Total = Rate EtOH + Rate EtOAc

**The numbers in parenthesis indicate the atomic ratio of Pd:Zn.

***Catalyst was prepared by treating $SiO_2$ with $NH_4OH$ and $Pd(NH_3)_4Cl_2$ (pH = 11.0) for 80 minutes, filtering the solid material from solution, rinsing with water, and drying in air at 120°C for 24 hours. Zinc was added by dissolving an appropriate amount of zinc nitrate in a small amount of water and adding this to the $Pd/SiO_2$ sample. The resulting solid was dried at 90°C in air and then calcined at 200°C for three hours prior to evaluation for catalytic activity.

The results set forth in Table III demonstrate that supporting palladium and zinc on silica results in catalysts with performance superior to the corresponding supported monometallic samples.

C. Catalysts Employed Using Ion-Exchange Technique

A series of catalysts similar to those described in Example 1b were prepared except that the Pd was added using an ion exchange technique and the Pd loading level was 2.8 wt %. The catalysts were prepared by treating the $SiO_2$ sample with $NH_4OH$ and $Pd(NH_3)_4Cl_2$ (pH = 11.0) for 80 minutes, filtering the solid material from solution, rinsing with water, and drying at 120 °C for 24 hours. Addition of zinc was accomplished according to the procedure of Example 1. The resulting samples were calcined for about 3 hours at about 200 °C prior to evaluation for catalytic activity. Results from this catalytic evaluation using the conditions employed in Example 2 are presented in Table IV.

Table IV

| Methyl Acetate Hydrogenation | | | | |
|---|---|---|---|---|
| Catalyst | Rate (µmole/g-cat sec) | | | |
| | MeOH | EtOH | EtOAc | Total* |
| Pd-Zn (1:0) | 3.17 | 0.02 | 0.86 | 0.89 |
| Pd-Zn (1:1) | 5.70 | 3.65 | 1.35 | 5.00 |
| Pd-Zn (1:2) | 8.72 | 3.73 | 3.05 | 6.78 |
| Pd-Zn (1:5) | 4.60 | 0.41 | 2.18 | 2.59 |
| Pd-Zn (0:1) | 0.00 | 0.00 | 0.00 | 0.00 |
| MeOH = Methanol, EtOH = Ethanol, EtOAc = Ethyl Acetate | | | | |

*Total = Rate EtOH + Rate EtOAc

The results set forth in Table IV indicate that catalysts prepared by ion exchange of the palladium component exhibit activity for the low pressure hydrogenation of carbonyl compounds. In addition, it is evident that catalyst performance is dependent on the Pd-Zn ratio. At lower Pd-Zn ratios, selectivity to the alcohol is enhanced, but the overall rate of hydrogenation is lower than observed at higher Pd-Zn ratios. Thus, the Pd-Zn ratio can be used to adjust the rate of hydrogenation.

D. Effect of Varied Pd Loading Level

A series of catalysts were prepared using the procedure described in Example 3c except that the palladium loading level was varied from 1-5 wt % and the Pd-Zn atomic ratio was held constant at 1:2. These catalysts were employed for the vapour phase hydrogenation of methyl acetate. All evaluations were conducted using a hydrogen/ester ratio of 4.0 and a gas hourly space velocity (GHSV; volume of gas/volume of catalyst/hr) of 30,180 $hr^{-1}$. For comparison, results from a 1% Pd on ZnO supported catalyst described in Example 1a are shown. Data from this latter catalyst were collected under identical conditions except that the total pressure was 730 psig (5110 kPa).

Table V

| Methyl Acetate Hydrogenation | | | | |
|---|---|---|---|---|
| Catalyst | Rate (µmole/g-cat sec) | | | |
| | MeOH | EtOH | EtOAc | Total* |
| 1% Pd-Zn/$SiO_2$ | 4.2 | 0.8 | 1.8 | 2.6 |
| 2.8% Pd-Zn/$SiO_2$ | 8.7 | 3.7 | 3.1 | 6.8 |
| 5% Pd-Zn/$SiO_2$ | 30.4 | 6.0 | 14.5 | 20.5 |
| 1% Pd/ZnO | 7.3 | 2.0 | 3.6 | 5.6 |
| MeOH = Methanol, EtOH = Ethanol, EtOAc = Ethyl Acetate | | | | |

*Total = Rate EtOH + Rate EtOAc

These results demonstrate that higher palladium loading levels give catalysts having dramatically higher activity for ester hydrogenation.

E. Effect of Water Addition on Supported Pd-Zn Catalyst

Catalyst prepared as described in Example 3d and containing 5 weight % Pd was evaluated as described in Example 3b, except the ester feed (methyl acetate) was supplemented with 1 weight % (based on the total weight of liquid feed) of water. The results of this experiment and a comparison in which no water was added to the feed are summarized in Table VII.

Table VII

| Effect of Water on Methyl Acetate Hydrogenation | | | | |
|---|---|---|---|---|
| Weight % Water in Feed | Rate of Formation, µmoles/g-cat sec | | | |
| | MeOH | EtOH | EtOAc | Total |
| 0.0 | 30.0 | 5.0 | 14.0 | 19.0 |
| 1.0 | 2.0 | 1.8 | 1.2 | 3.0 |

In the absence of water, transesterification product, ethyl acetate, represents more than 70% (14 out of 19 moles) of the reaction product; while in the presence of water, transesterification product represents only about 40% of the reaction product. Thus, the presence of water is seen to supress transesterification and thereby give improved selectivity to the desired direct hydrogenation product.

EXAMPLE 4 - Hydrogenation of Methl Propionate

b. Supported Catalyst Prepared as Described in Example 1b:

The catalyst prepared as described in Example 3d and containing 5 weight % was used for the hydrogenation of methyl propionate to produce n-propanol, employing the procedure set forth in Example 3b, except that methyl propionate was used instead of methyl acetate.

The conversion of methyl propionate to n-propanol proceeded at a rate of about 14 µmoles/g-cat sec, thereby demonstrating that Pd-Zn/$SiO_2$ catalyst is effective for the hydrogenation of methyl propionate.

EXAMPLE 7 - Hydrogenation of Ethyl Acetate

b. Supported Catalyst Prepared as Described in Example 1b:

The catalyst prepared as described in Example 3d and containing 5 weight % Pd was used for the hydrogenation of ethyl acetate according to the procedure set forth in Example 3b, except that ethyl acetate was used instead of methyl acetate.

The conversion of ethyl acetate to ethanol proceeded at a rate of about 30 µmoles/g-cat sec, thereby demonstrating that Pd-Zn/$SiO_2$ is an effective catalyst for the hydrogenation of ethyl acetate.

EXAMPLE 12 - Methyl Oleate Hydrogenation

b. Supported Catalyst Prepared as Described in Example 1b:

Fifty cc's of a 5% Pd (Pd-Zn ratio of 1:2) on $SiO_2$ catalyst (prepared as described in Example 3d was loaded into a 1" tubular reactor. After catalyst was pre-treated at 1200 psig (8400 kPa) at 300 °C in a flowing hydrogen atmosphere for 12 hours, methyl oleate feed was pumped into the tubular reactor (maintained at 300 °C) at the rate of about 1.4 mL/minute with a hydrogen flow of about 744 standard cubic centimeters per minute.

Under the above conditions, a 9% conversion of methyl oleate was obtained with nearly quantitative selectivity to a 2:1 mixture of oleyl alcohol and stearyl alcohol.

## Claims

**1.** A method for preparing high activity, low pressure hydrogenation catalysts comprising palladium and zinc on a support, said method comprising :

(a) contacting said support with at least one of zinc or a reducible compound thereof and palladium or a reducible compound thereof ;

(b) optionally calcining the resulting treated support obtained in step (a) in the presence of an oxygen-containing gas at a temperature in the range of 200 up to 400 °C for a time sufficient to activate said palladium and/or said zinc component ;

(c) where palladium is employed in step (a), contacting said palladium-treated support with a reducing atmosphere under conditions sufficient to cause reduction of at least a portion of the

palladium to less than the +2 oxidation state ;
(d) contacting said support again with at least one member selected from the group consisting of palladium or a reducible compound thereof and zinc or a reducible compound thereof, with the proviso that said support is ultimately contacted with both palladium and zinc ;
(e) optionally calcining the resulting palladium and zinc treated support in the presence of an oxygen-containing gas at a temperature in the range of 200° up to 400°C for a time sufficient to activate said palladium and said zinc component; and thereafter
(f) contacting the zinc and palladium-treated support with a reducing atmosphere under conditions sufficient to cause reduction of at least a portion of the palladium to less than the +2 oxidation state.

**2.** A method in accordance with Claim 1 wherein said contacting step (a) comprises contacting said support with palladium or a reducible compound thereof; wherein optional step (b) is omitted; wherein step (c) comprises contacting said palladium-treated support with a reducing atmosphere under conditions sufficient to form highly dispersed, supported zerovalent palladium; wherein optional step (d) comprises contacting said supported zerovalent palladium with zinc or a reducible compound thereof; and thereafter; optionally carrying out steps (e) and (f).

**3.** A method in accordance with Claim 1 wherein the quantity of said palladium employed is in the range of 0.01 up to 20 wt %, calculated as the metal and based on the total weight of palladium, zinc and support.

**4.** A method in accordance with Claim 3 wherein the atomic ratio of Pd to Zn falls within the range of 0.01 up to 10.

**5.** A method in accordance with Claim 1 wherein said support has a surface area of at least 1 $m^2/g$.

**6.** A method in accordance with Claim 5 wherein said support is selected from the group consisting of:
$SiO_2$
$TiO_2$
C,
Rare earth oxides,
$Al_2O_3$,
as well as mixtures of any two or more thereof.

**7.** A method in accordance with Claim 5 wherein said support is silica.

**8.** A method in accordance with Claim 7 wherein the quantity of palladium employed is in the range of 0.5 Up to 6 weight %, calculated as the metal and based on the total weight of palladium, zinc, and support.

**9.** A method in accordance with Claim 8 wherein the atomic ratio of Pd to Zn falls within the range of 0.2 up to 5.0.

**10.** A method in accordance with Claim 1 wherein said calcination is carried out for a time in the range of 0.5 up to 8 hours.

**11.** A method in accordance with Claim 2 wherein said reducing atmosphere comprises active hydrogen.

**12.** A method in accordance with Claim 11 wherein said contacting with a reducing atmosphere is carried out at a temperature in the range of 25 up to 400°C for a time sufficient to form highly dispersed, supported zerovalent palladium.

**13.** A method in accordance with Claim 1 wherein said reducing atmosphere comprises active hydrogen.

**14.** A method in accordance with Claim 13 wherein said contacting with a reducing atmosphere is carried out at a temperature in the range of 25 up to 400°C for a time sufficient to reduce at least a portion of the palladium to less than the +2 oxidation state.

**15.** A process for the low pressure hydrogenation of carbonyl-containing compounds to produce the corresponding alcohol, wherein said carbonyl-containing compounds have the structure:

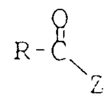

wherein R is a $C_1$ -$C_{20}$ alkyl or substituted alkyl radical; or
a $C_2$-$C_{20}$ alkenyl or alkynyl radical or a substituted derivative thereof;
wherein said substituted groups include ethers, amines, additional carbonyl groups, aryl groups, hydroxyl groups and alkoxy groups; and
Z = H,
R', wherein R' is defined, the same as R, and is selected independently of R,
OR', wherein R' is as defined above,
X, wherein X is any one of the halogens, or
$NR''_2$, wherein each R'' is independently selected from H or R';
with the proviso that R and Z can be joined as part of a polymethylene or hydrocarbyl- or heteroatom-substituted polymethylene radical;
said process comprising contacting said carbonyl-containing compounds or mixtures of any two or more thereof with a supported palladium and zinc containing catalyst as prepared according to the process of any of claims 1 - 14; wherein said contacting is carried out in the presence of hydrogen under hydrogenation conditions.

**16.** A process in accordance with Claim 15 wherein said contacting is carried out in the further presence of in the range of 0.01 up to 2.0 wt % water, based on the total weight of reactants and solvent charged to the reactor.

**17.** A process in accordance with Claim 15 wherein said hydrogenation conditions comprise a temperature in the range of 25 up to 400 ° C, and a pressure in the range of 100 up to 10,000 psig (700 up to 70000 kPa).

**18.** A process in accordance with Claim 15 wherein the hydrogen partial pressure falls within the range of 100 up to 10,000 psig (700 up to 70000 kPa).

**19.** A process in accordance with Claim 15 wherein said hydrogenation conditions comprise a temperature in the range of 100 up to 290 ° C and a pressure in the range of 100 up to 2500 psig (700 up to 17500 kPa).

**20.** A process in accordance with Claim 15 wherein the liquid hourly space velocity falls within the range of about 0.01 up to 100 $h^{-1}$.

**21.** A process in accordance with Claim 15 wherein the weight ratio of carbonyl-containing compound to catalyst falls within the range of 1:1 up to 10,000:1.

**22.** A process in accordance with Claim 15 wherein the carbonyl-containing compound is selected from the group consisting of:

(a) $YO_2C—A—CO_2Y$ ,

wherein A is an alkylene moiety, an alkenylene moiety, or an alkynylene moiety having 1 up to 20 carbon atoms, or substituted derivative thereof, or a cycloalkyl or cycloalkenyl moiety having 4-12 carbon atoms or substituted derivative thereof; and wherein each Y is independently a $C_1$ up to $C_{12}$ alkyl, alkenyl or alkynyl radical or substituted derivative thereof;

(b) $B—CO_2Y$

wherein B is an alkyl, alkenyl or alkynyl radical, or substituted derivative thereof, having 1 up to 20 carbon atoms; and
wherein Y is defined as above;

(c) 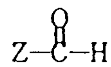

wherein Z is an alkyl, alkenyl or alkynyl radical having 1 up to 20 carbon atoms or substituted derivatives thereof; and
mixtures of any two or more thereof.

**23.** A process in accordance with Claim 22 wherein the carbonyl-containing compound comprises a dialkyl adipate.

**24.** A process in accordance with Claim 22 wherein the carbonyl-containing compound comprises a dialkyl cyclohexanedicarboxylate.

**25.** A process in accordance with Claim 22 wherein the carbonyl-containing compound is selected from the group consisting of an alkyl oleate, an alkyl stearate, an alkyl linoleate, an alkyl linolenate, an alkyl $\alpha$-eleostearate, an alkyl $\beta$-eleostearate, and mixtures of any two or more thereof.

**26.** A process in accordance with Claims 22 wherein the carbonyl-containing compound comprises a dialkyl butanedicarboxylate.

**27.** A process in accordance with Claims 22 wherein the carbonyl-containing compound comprises a glycerol ester.

**28.** A process in accordance with Claim 22 wherein the carbonyl-containing compound comprises a dialkyl glutarate.

**29.** A process in accordance with Claims 22 wherein the carbonyl-containing compound is selected from the group consisting of dialkyl fumarates, succinates, maleates, and mixtures of any two or more thereof.

**30.** A process in accordance with Claim 22 wherein the carbonyl-containing compound comprises an alkyl decanoate.

**31.** A process in accordance with Claim 22 wherein the carbonyl-containing compound comprises an alkyl dodecanoate.

**32.** A process in accordance with Claim 22 wherein the carbonyl-containing compound is selected from the group consisting of alkyl acetates, propionates, butyrates, valerates, caproates, and mixtures of any two or more thereof.

**33.** A process in accordance with any of claims 23 - 32 wherein said alkyl radical has in the range of 1 up to 6 carbon atoms.

## Patentansprüche

**1.** Verfahren zur Herstellung von hochaktiven Niederdruck-Hydrierungskatalysatoren mit Palladium und Zink auf einem Träger, das umfaßt:
(a) Kontaktieren des Trägers mit mindestens einer der Komponenten Zink oder einer reduzierbaren Verbindung hiervon sowie Palladium oder einer reduzierbaren Verbindung hiervon;
(b) gegebenenfalls Calcinieren des gemäß Stufe (a) erhaltenen behandelten Trägers in Gegenwart eines Sauerstoff enthaltenden Gases bei einer Temperatur im Bereich von 200 bis zu 400 °C eine solche Zeitspanne lang, die ausreicht, um die Palladium- und/oder die Zinkkomponente zu aktivieren;

(c) in dem Falle, in dem in Stufe (a) Palladium verwendet wird, Kontaktieren des Palladium-behandelten Trägers mit einer reduzierenden Atmosphäre unter Bedingungen, die ausreichen, um eine Reduktion mindestens eines Teiles des Palladiums in einen geringeren als den +2 Oxidationszustand zu bewirken;

(d) erneutes Kontaktieren des Trägers mit mindestens einem Glied ausgewählt aus der Gruppe bestehend aus Palladium oder einer reduzierbaren Verbindung hiervon und Zink oder einer reduzierbaren Verbindung hiervon, wobei gilt, daß der Träger letzten Endes mit sowohl Palladium als auch Zink kontaktiert wurde;

(e) gegebenenfalls Calcinieren des erhaltenen Palladium- und Zink-behandelten Trägers in Gegenwart eines Sauerstoff enthaltenden Gases bei einer Temperatur im Bereich von 200° bis zu 400°C eine solche Zeitspanne lang, die ausreicht, um die Palladium- und die Zinkkomponente zu aktivieren und daraufhin

(f) Kontaktieren des Zink- und Palladium-behandelten Trägers mit einer reduzierenden Atmosphäre unter Bedingungen, die ausreichen, um eine Reduktion mindestens eines Teiles des Palladiums in einen geringeren als den +2 Oxidationszustand zu bewirken.

**2.** Verfahren nach Anspruch 1, bei dem die Kontaktierungsstufe (a) ein Kontaktieren des Trägers mit Palladium oder einer reduzierbaren Verbindung hiervon umfaßt; bei dem die gegebenenfalls anzuwendende Stufe (b) weggelassen wird; bei dem die Stufe (c) ein Kontaktieren des Palladium-behandelten Trägers mit einer reduzierenden Atmosphäre unter Bedingungen umfaßt, die ausreichen, um hochdisperses, auf einem Träger befindliches Null-wertiges Palladium zu erzeugen;
bei dem die gegebenenfalls durchzuführende Stufe (d) ein Kontaktieren des auf einem Träger befindlichen Null-wertigen Palladiums mit Zink oder einer reduzierbaren Verbindung hiervon umfaßt; und bei dem danach gegebenenfalls die Stufen (e) und (f) durchgeführt werden.

**3.** Verfahren nach Anspruch 1, bei dem die Menge des verwendeten Palladiums im Bereich von 0,01 bis zu 20 Gew.-%, berechnet als Metall und bezogen auf das Gesamtgewicht von Palladium, Zink und Träger liegt.

**4.** Verfahren nach Anspruch 3, bei dem das Atomverhältnis von Pd zu Zn in den Bereich von 0,01 bis zu 10 fällt.

**5.** Verfahren nach Anspruch 1, bei dem der Träger einen Oberflächenbereich von mindestens 1 $m^2/g$ aufweist.

**6.** Verfahren nach Anspruch 5, bei dem der Träger ausgewählt ist aus der Gruppe bestehend aus:
$SiO_2$
$TiO_2$
C,
Oxiden der seltenen Erdmetalle,
$Al_2O_3$,
wie auch Mischungen von beliebigen zwei oder mehreren Komponenten hiervon.

**7.** Verfahren nach Anspruch 5, bei dem der Träger ein Kieselsäureträger ist.

**8.** Verfahren nach Anspruch 7, bei dem die Menge an verwendetem Palladium im Bereich von 0,5 bis zu 6 Gew.-%, berechnet als das Metall und bezogen auf das Gesamtgewicht von Palladium, Zink und Träger liegt.

**9.** Verfahren nach Anspruch 8, bei dem das Atomverhältnis von Pd zu Zn in den Bereich von 0,2 bis zu 5,0 fällt.

**10.** Verfahren nach Anspruch 1, bei dem die Calcinierung 0,5 bis zu 8 Stunden lang durchgeführt wird.

**11.** Verfahren nach Anspruch 2, bei dem die reduzierende Atmosphäre aktiven Wasserstoff enthält.

**12.** Verfahren nach Anspruch 11, bei dem das Kontaktieren mit einer reduzierenden Atmosphäre bei einer Temperatur im Bereich von 25 bis zu 400°C eine solche Zeitspanne lang erfolgt, die ausreicht, um

hochdisperses, auf einem Träger befindliches Null-wertiges Palladium zu erzeugen.

**13.** Verfahren nach Anspruch 1, bei dem die reduzierende Atmosphäre aktiven Wasserstoff enthält.

**14.** Verfahren nach Anspruch 13, bei dem das Kontaktieren mit einer reduzierenden Atmosphäre bei einer Temperatur im Bereich von 25 bis zu 400 °C eine solche Zeitspanne lang durchgeführt wird, die ausreicht, um mindestens einen Teil des Palladiums auf einen geringeren als den +2 Oxidationszustand zu reduzieren.

**15.** Verfahren zur Niederdruck-Hydrierung von Carbonyl enthaltenden Verbindungen unter Erzeugung des entsprechenden Alkohols, bei dem die Carbonyl enthaltenden Verbindungen der folgenden Struktur entsprechen:

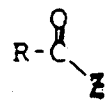

worin R ein $C_1$-$C_{20}$-Alkylrest oder substituierter Alkylrest ist; oder
ein $C_2$-$C_{20}$-Alkenyl- oder-Alkynylrest oder ein substituiertes Derivat hiervon;
wobei zu den substituierten Derivaten gehören Ether-, Amin-, zusätzliche Carbonyl-, Aryl-, Hydroxyl- und Alkoxygruppen; und
Z = H ist oder R', worin R' die gleiche Bedeutung hat wie R und unabhängig von R ausgewählt ist,
OR', worin R' die angegebene Bedeutung hat,
X, worin X ein beliebiges Halogen ist oder
$NR''_2$, worin R'' unabhängig voneinander ausgewählt ist von H oder R';
wobei gilt, daß R und Z miteinander verbunden sein können, als Teil eines Polymethylen- oder Hydrocarbyl- oder Heteroatom-substituierten Polymethylenrestes;
wobei das Verfahren das Kontaktieren der Carbonyl enthaltenden Verbindungen oder Mischungen von beliebigen zwei oder mehreren hiervon mit einem Palladium- und Zink enthaltenden Trägerkatalysators umfaßt, der nach dem Verfahren eines der Ansprüche 1 bis 14 hergestellt worden ist, wobei das Kontaktieren in Gegenwart von Wasserstoff unter Hydrierungsbedingungen durchgeführt wird.

**16.** Verfahren nach Anspruch 15, bei dem das Kontaktieren durchgeführt wird in weiterer Gegenwart von 0,01 bis zu 2,0 Gew.-% Wasser, bezogen auf das Gesamtgewicht der Reaktionskomponenten und des Lösungsmittels, die in den Reaktor eingespeist werden.

**17.** Verfahren nach Anspruch 15, bei dem die Hydrierungsbedingungen eine Temperatur im Bereich von 25 bis zu 400 °C und einen Druck im Bereich von 700 bis zu 70000 kPa umfassen.

**18.** Verfahren nach Anspruch 15, bei dem der Wasserstoff-Partialdruck in den Bereich von 700 bis zu 70000 kPa fällt.

**19.** Verfahren nach Anspruch 15, bei dem die Hydrierungsbedingungen eine Temperatur im Bereich von 100 bis zu 290 °C und einen Druck im Bereich von 700 bis zu 17500 kPa umfassen.

**20.** Verfahren nach Anspruch 15, bei dem die stündliche flüssige Raumgeschwindigkeit in den Bereich von etwa 0,01 bis zu 100 $h^{-1}$ fällt.

**21.** Verfahren nach Anspruch 15, bei dem das Gewichtsverhältnis von Carbonyl enthaltender Verbindung zu Katalysator in den Bereich von 1:1 bis zu 10000:1 fällt.

**22.** Verfahren nach Anspruch 15, bei dem die Carbonyl enthaltende Verbindung ausgewählt ist aus der Gruppe bestehend aus:

(a) $YO_2C—A—CO_2Y$ ,

worin A steht für einen Alkylenrest, einen Alkenylenrest oder einen Alkynylenrest mit 1 bis zu 20

Kohlenstoffatomen oder ein substituiertes Derivat hiervon oder einen Cycloalkyl- oder Cycloalkenylrest mit 4 bis 12 Kohlenstoffatomen oder ein substituiertes Derivat hiervon; wobei Y jeweils unabhängig voneinander steht für einen $C_1$ bis zu $C_{12}$ Alkyl-, Alkenyl- oder Alkynylrest oder ein substituiertes Derivat hiervon;

(b) $B\text{-}CO_2Y$

worin B steht für einen Alkyl-, Alkenyl- oder Alkynylrest oder ein substituiertes Derivat hiervon mit 1 bis zu 20 Kohlenstoffatomen und
worin Y wie oben angegeben definiert ist;

(c) 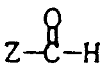

worin Z steht für einen Alkyl-, Alkenyl- oder Alkynylrest mit 1 bis zu 20 Kohlenstoffatomen oder substituierte Derivate hiervon; und
Mischungen von beliebigen zwei oder mehreren hiervon.

**23.** Verfahren nach Anspruch 22, bei dem die Carbonyl enthaltende Verbindung ein Dialkyladipat umfaßt.

**24.** Verfahren nach Anspruch 22, bei dem die Carbonyl enthaltende Verbindung ein Dialkylcyclohexandicarboxylat umfaßt.

**25.** Verfahren nach Anspruch 22, bei dem die Carbonyl enthaltende Verbindung ausgewählt ist aus der Gruppe bestehend aus einem Alkyloleat, einem Alkylstearat, einem Alkyllinoleat, einem Alkyllinolenat, einem Alkyl-$\alpha$-eleostearat, einem Alkyl-$\beta$-eleostearat und Mischungen von beliebigen zwei oder mehreren hiervon.

**26.** Verfahren nach Anspruch 22, bei dem die Carbonyl enthaltende Verbindung ein Dialkylbutandicarboxylat umfaßt.

**27.** Verfahren nach Anspruch 22, bei dem die Carbonyl enthaltende Verbindung einen Glyzerinester umfaßt.

**28.** Verfahren nach Anspruch 22, bei dem die Carbonyl enthaltende Verbindung ein Dialkylglutarat umfaßt.

**29.** Verfahren nach Anspruch 22, bei dem die Carbonyl enthaltende Verbindung ausgewählt ist aus der Gruppe bestehend aus Dialkylfumaraten, -succinaten, -maleaten und Mischungen von beliebigen zwei oder mehreren hiervon.

**30.** Verfahren nach Anspruch 22, bei dem die Carbonyl enthaltende Verbindung ein Alkyldecanoat umfaßt.

**31.** Verfahren nach Anspruch 22, bei dem die Carbonyl enthaltende Verbindung ein Alkyldodecanoat umfaßt.

**32.** Verfahren nach Anspruch 22, bei dem die Carbonyl enthaltende Verbindung ausgewählt ist aus der Gruppe bestehend aus Alkylacetaten, -propionaten, -butyraten, -valeraten, -caproaten sowie Mischungen von beliebigen zwei oder mehreren hiervon.

**33.** Verfahren nach einem der Ansprüche 23 bis 32, bei dem der Alkylrest im Bereich von 1 bis zu 6 Kohlenstoffatomen liegt.

## Revendications

**1.** Procédé pour préparer des catalyseurs d'hydrogénation sous basse pression d'activité élevée comprenant du palladium et du zinc sur un support, ledit procédé comprenant :

(a) la mise en contact dudit support avec au moins un composant choisi parmi le zinc ou un de ses composés réductibles et le palladium ou un de ses composés réductibles;
(b) facultativement la calcination du support traité résultant obtenu à l'étape (a) en présence d'un gaz contenant de l'oxygène à une température dans la gamme de 200 à 400 °C pendant une durée suffisante pour activer ledit composant de palladium et/ou ledit composant de zinc;
(c) lorsque le palladium est utilisé dans l'étape (a), la mise en contact du support traité par le palladium avec une atmosphère réductrice dans des conditions suffisantes pour provoquer la réduction d'au moins une portion du palladium au-dessous du degré d'oxydation +2;
(d) la mise en contact dudit support à nouveau avec au moins un composant choisi parmi le palladium ou un de ses composés réductibles et le zinc ou un de ses composés réductibles, avec la condition que ledit support soit finalement mis en contact à la fois avec le palladium et le zinc;
(e) facultativement la calcination du support résultant traité par le palladium et le zinc en présence d'un gaz comprenant de l'oxygène à une température dans la gamme de 200 à 400 °C pendant une durée suffisante pour activer ledit composant de palladium et ledit composant de zinc; et ensuite
(f) la mise en contact du support traité par le zinc et le palladium avec une atmosphère réductrice dans des conditions suffisantes pour provoquer la réduction d'au moins une portion du palladium au-dessous du degré d'oxydation +2.

**2.** Procédé selon la revendication 1, dans lequel ladite étape de mise en contact (a) comprend la mise en contact dudit support avec le palladium ou un de ses composés réductibles; dans lequel l'étape facultative (b) est supprimée ; dans lequel l'étape (c) comprend la mise en contact dudit support traité par le palladium avec une atmosphère réductrice dans des conditions suffisantes pour former du palladium de valence 0 fortement dispersé sur support ; dans lequel l'étape facultative (d) comprend la mise en contact dudit palladium de valence 0 sur support avec le zinc ou un de ses composés réductibles ; et ensuite facultativement la mise en oeuvre des étapes (e) et (f).

**3.** Procédé selon la revendication 1, dans lequel la quantité dudit palladium utilisée est dans la gamme de 0,01 à 20 % en poids, calculée en métal par rapport au poids total du palladium, du zinc et du support.

**4.** Procédé selon la revendication 3, dans lequel le rapport atomique de Pd à Zn est dans la gamme de 0,01 à 10.

**5.** Procédé selon la revendication 1, dans lequel ledit support a une surface spécifique d'au moins 1 $m^2/g$.

**6.** Procédé selon la revendication 5, dans lequel ledit support est choisi parmi :
$SiO_2$
$TiO_2$
C,
les oxydes des terres rares,
$Al_2O_3$ et
les mélanges de deux ou plusieurs quelconques d'entre eux.

**7.** Procédé selon la revendication 5, dans lequel ledit support est la silice.

**8.** Procédé selon la revendication 7, dans lequel la quantité de palladium utilisée est dans la gamme de 0,5 à 6 % en poids, calculée en métal par rapport au poids total de palladium, de zinc et de support.

**9.** Procédé selon la revendication 8, dans lequel le rapport atomique de Pd à Zn est dans la gamme de 0,2 à 5,0.

**10.** Procédé selon la revendication 1, dans lequel ladite calcination est mise en oeuvre pendant une durée dans la gamme de 0,5 à 8 h.

**11.** Procédé selon la revendication 2, dans lequel ladite atmosphère réductrice comprend de l'hydrogène actif.

**12.** Procédé selon la revendication 11, dans lequel ladite mise en contact avec une atmosphère réductrice est mise en oeuvre à une température dans la gamme de 25 à 400 °C pendant une durée suffisante

pour former du palladium de valence 0 fortement dispersé sur support.

**13.** Procédé selon la revendication 1, dans lequel ladite atmosphère réductrice comprend de l'hydrogène actif.

**14.** Procède selon la revendication 13, dans lequel ladite mise en contact avec une atmosphère réductrice est mise en oeuvre à une température dans la gamme de 25 à 400 °C pendant une durée suffisante pour réduire au moins une portion du palladium au-dessous du degré d'oxydation +2.

**15.** Procédé pour l'hydrogénation sous basse pression de composés carbonylés pour produire l'alcool correspondant, dans lequel lesdits composés carbonylés répondent à la formule

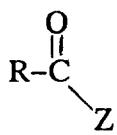

dans laquelle R est un groupe alkyle ou alkyle substitué en $C_1$-$C_{20}$ ; ou un radical alcényle ou alcynyle en $C_2$-$C_{20}$ ou un de leurs dérivés substitués; dans lequel lesdits groupes substitués comprennent les éthers, les amines, les groupes carbonyles supplémentaires, les groupes aryles, les groupes hydroxyles et les groupes alcoxy ; et

Z = H,

R', qui est défini comme R et est choisi indépendamment de R,

OR' dans lequel R' est défini comme ci-dessus,

X qui est l'un quelconque des halogènes ou

NR'' 2 dans lequel chaque R'' est choisi indépendamment entre H et R';

avec la condition que R et Z peuvent être reliés comme partie d'un radical polyméthylène ou d'un radical polyméthylène substitué par un hydrocarbyle ou un hétéroatome;

ledit procédé comprenant la mise en contact desdits composés carbonylés ou desdits mélanges de deux ou plusieurs quelconques d'entre eux avec un catalyseur sur support contenant du palladium et du zinc préparé par le procédé selon l'une quelconque des revendications 1-14; dans lequel ladite mise en contact est mise en oeuvre en présence d'hydrogène dans des conditions d'hydrogénation.

**16.** Procédé selon la revendication 15, dans lequel ladite mise en contact est mise en oeuvre en outre en présence de 0,01 à 2,0 % en poids d'eau, par rapport au poids total des réactifs et du solvant chargés dans le réacteur.

**17.** Procédé selon la revendication 15, dans lequel lesdites conditions d'hydrogénation comprennent une température dans la gamme de 25 à 400 °C et une pression manométrique de 100 à 10000 psig (700 à 70000 kPa).

**18.** Procédé selon la revendication 15, dans lequel la pression partielle d'hydrogène est dans la gamme de 100 à 10000 psig (700 à 70000 kPa).

**19.** Procédé selon la revendication 15, dans lequel lesdites conditions d'hydrogénation comprennent une température dans la gamme de 100 à 290 °C et une pression dans la gamme de 100 à 2500 psig (700 à 17500 kPa).

**20.** Procédé selon la revendication 15, dans lequel la vitesse spatiale horaire du liquide est dans la gamme d'environ 0,01 à 100 $h^{-1}$.

**21.** Procédé selon la revendication 15, dans lequel le rapport pondéral du composé carbonylé au catalyseur est dans la gamme de 1:1 à 10000:1.

**22.** Procédé selon la revendication 15, dans lequel le composé carbonylé est choisi parmi les suivants :

(a) $YO_2C—A—CO_2Y$,

où A est un reste alkylène, un reste alcénylène ou un reste alcynylène en $C_1$-$C_{20}$ ou un de leurs dérivés substitués, ou un reste cycloalkyle ou cycloalcényle en $C_4$-$C_{12}$ ou un de leurs dérivés substitués ; et où chaque Y est indépendamment un radical alkyle, alcényle ou alcynyle en $C_1$-$C_{12}$ ou un de leurs dérivés substitués;

(b) $B$-$CO_2Y$

où B est un radical alkyle, alcényle ou alcynyle ou un de leurs dérivés substitués, en $C_1$-$C_{20}$ ; et où Y est défini comme ci-dessus;

(c) 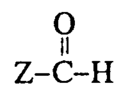

dans lesquels Z est un radical alkyle, alcényle ou alcynyle en $C_1$-$C_{20}$ ou un de leurs dérivés substitués ; et
des mélanges de deux ou plusieurs quelconques d'entre eux.

**23.** Procédé selon la revendication 22 dans lequel le composé carbonylé comprend un adipate de dialkyle.

**24.** Procédé selon la revendication 22, dans lequel le composé carbonylé comprend un cyclohexanedicarboxylate de dialkyle.

**25.** Procédé selon la revendication 22, dans lequel le composé carbonylé est choisi parmi un oléate d'alkyle, un stéarate d'alkyle, un linoléate d'alkyle, un linolénate d'alkyle un $\alpha$-éléostéarate d'alkyle, un $\beta$-éléostéarate d'alkyle et les mélanges de deux ou plusieurs quelconques d'entre eux.

**26.** Procédé selon la revendication 22, dans lequel le composé carbonylé comprend un butanedicarboxylate de dialkyle.

**27.** Procédé selon la revendication 22, dans lequel le composé carbonylé comprend un ester de glycérol.

**28.** Procédé selon la revendication 22, dans lequel le composé carbonylé comprend un glutarate de dialkyle.

**29.** Procédé selon la revendication 22, dans lequel le composé carbonylé est choisi parmi les fumarates, les succinates, et les maléates de dialkyle et les mélanges de deux ou plusieurs quelconques d'entre eux.

**30.** Procédé selon la revendication 22, dans lequel le composé carbonylé comprend un décanoate d'alkyle.

**31.** Procédé selon la revendication 22, dans lequel le composé carbonylé comprend un dodécanoate d'alkyle.

**32.** Procédé selon la revendication 22, dans lequel le composé carbonylé est choisi parmi les acétates, propionates, butyrates, valérates et caproates d'alkyles et les mélanges de deux ou plusieurs quelconques d'entre eux.

**33.** Procédé selon l'une quelconque des revendications 23 à 32, dans lequel ledit radical alkyle est dans la gamme de $C_1$-$C_6$.